# EUROPEAN PATENT APPLICATION

(11) **EP 0 993 936 A2**
(43) Date of publication of application: **19.04.2000**
(21) Application number: 99119964.7
(22) Date of filing: 12.10.1999
(51) Int. Cl.: B32B 7/02, A61K 7/48

(54) **Gel sheet for cosmetics and method for producing the same**

(30) Priority: 13.10.1998 JP 29108098; 13.10.1998 JP 29108198
(71) Applicant: NITTO DENKO CORPORATION, Ibaraki-shi, Osaka 567-8680 (JP)
(72) Inventor: Konno, Masayuki, c/o Nitto Denko Corporation, Ibaraki-shi, Osaka 567-8680 (JP); Nakagawa, Takeaki, c/o Nitto Denko Corporation, Ibaraki-shi, Osaka 567-8680 (JP); Ujiie, Takako, c/o Nitto Denko Corporation, Ibaraki-shi, Osaka 567-8680 (JP); Kawaski, Takashi, c/o Nitto Denko Corporation, Ibaraki-shi, Osaka 567-8680 (JP)
(74) Representative: Forstmeyer, Dietmar, Dr. rer. nat., Dipl.-Chem.

(57) **Abstract**

Disclosed is a gel sheet for cosmetics, comprising a sheet-like substrate having laminated thereon a hydrous gel sheet, in which the substrate comprises a gel non-impregnating layer and a gel impregnating layer and wherein said hydrous gel layer is provided on the gel non-impregnating layer. In a preferred embodiment, the gel sheet for cosmetics is produced by laminating a hydrous gel layer comprising polyacrylic acid or its salts, water, glycerin, etc. on a substrate comprising a transparent film laminated on a fiber layer, for example, polyethylene film/pulp (paper), polyester film/rayon unwoven fabric, or the like, heating the resulting composite at room temperature or higher temperatures for aging. The gel sheet for cosmetics is excellent in a feeling upon application, interlocking with the substrate, but not causes strike through. The gel sheet in a preferred embodiment gives an impression of transparency and is unattractive when it is applied to the skin.

## Description

### BACKGROUND OF THE INVENTION

### FIELD OF THE INVENTION

The present invention relates to a gel sheet for cosmetics and to a method for producing the same. More particularly, it relates to a gel sheet for cosmetics having a hydrous gel in the form of a thin film coated on a backing layer and to a method for producing such a gel sheet.

### DESCRIPTION OF THE RELATED ART

As a gel sheet product for use in the field of cosmetics, a pack agent or the like to be applied to faces has heretofore been known. Recently, with an increasing attention to the effect of retaining moisture of the skin and giving a cool feeling when it is applied to the skin, attempts have been made to provide a wide variety of gel sheet products. In particular, in view of giving an improved feeling upon application to the skin surface and reduction in cost for transportation, there has been an increasing demand for a thinner gel sheet.

Gel sheet products generally are constituted by a substrate made of, for example, a plastic or unwoven fabric, and a hydrous gel layer containing a large amount of water, laminated on the substrate. The substrate commonly used is made of unwoven fabric since it is advantageous in that a portion of the hydrous gel is impregnated into the unwoven fabric to increase interlocking or anchorage between the gel layer and the substrate, thus leaving a less amount of residual gel on the skin after the gel sheet is peeled off from the skin. Furthermore, use of unwoven fabric provides a soft touch upon application on the skin. For hydrous gel layers, there have been generally used carboxyl group-containing polymers such as polyacrylic acid, which have been confirmed of safety. These are usually used after crosslinking with a polyvalent metal salt, for example, aluminum hydroxide.

Such a gel sheet can be prepared, for example, by the following method. A polymer selected from various polymers having a carboxyl group is dissolved or suspended in water or hydrophilic solvent such as an aqueous alcohol solution to form a liquid, which is coated on a separator film, by casting, for example, to form a liquid film. Thereafter, an aqueous solution of a polyvalent metal salt is sprayed or otherwise applied to the coated liquid to gel it to form a hydrous gel layer. Alternatively, the polyvalent metal salt is dissolved in the liquid of the polymer and the resulting mixture is coated on a separator film using a coater. The gel film thus obtained is transferred, as it is or after drying it to evaporate a certain amount of water, onto the substrate and wound into a roll. Finally, to complete the crosslinking, the roll is heated at around 50°C for a long time, usually for 3 days, to effect aging to obtain a gel sheet product.

However, in the above-described conventional method, the crosslinking with a polyvalent metal salt takes a long time so that if the sheet in the form of a roll is left to stand for sometime, the roll product receives a pressure in its inside, which causes the problem that the gel is impregnated into the substrate, with the result that the thickness of the gel layer fluctuates to an unnegligible extent. In particular, when it is attempted to form a hydrous gel layer in the form of a thin film, the fluctuation in thickness of the hydrous gel layer becomes considerably large.

Further, when unwoven fabric is used for constituting a substrate in order to increase a good feeling upon use, the hydrous gel layer, to the worst, penetrates the substrate to its back side, thus causing the problem of a so-called "strike through." In particular, attempts to use a thinner substrate in order to reduce the thickness of the hydrous gel sheet tend to cause severer strike thorough, which reduces yield of the hydrous gel sheet product greatly. Thus, the above method has problems in manufacture.

To solve these problems, various attempts have been made, which include adjustment of crosslinking rate, selection of an appropriate substrate and the like countermeasures. However, an increase in crosslinking rate, intended to shorten a crosslinking time, results in gelling of the material during the film formation to increase the viscosity. This results in a shortened pot life. As a result, no thin film can be obtained and when a conventional coater is used, the thickness of the hydrous gel layer fluctuates too much to efficiently obtain a hydrous gel layer in the form of a thin film.

On the other hand, if use is made of unwoven fabric having a large basis weight to cope with the strike through, there occurs the problem after application of the hydrous gel sheet to the skin that the backside of the substrate tends to be caught by a nail and peeled off. Furthermore, in such a case, users would be given a stiff or starchy touch and is compelled to realize the thickness of the sheet when it is applied to the skin. This causes the users to have rather an unpleasant feeling upon use of the sheet.

If a substrate in the form of a non-porous film is used by giving priority to the prevention of strike through, a hydrophobic substrate will cause the gel to remain on the skin surface upon peeling if the substrate is formed into a thin film since the hydrophobic substrate is poor in interlocking with the hydrous gel. For this reason, it is necessary to practice a primer treatment on the surface of substrate, which results in an increased cost for the gel sheet. On the other hand, a hydrophilic substrate have no such defect but the strength of substrate itself is decreased so that it cannot be used any longer. Thus, the problem of residual gel tended to occur easily.

Further, it would be considered possible that the formed gel film is heated and aged without being wound into a roll. However, for this method, a much broader space for operation must be secured when conducting thermal aging. Thus, the efficiency of work decreases to a greater extent and mass production is difficult.

On the other hand, although it is sure that a hydrous gel layer in the form of a thin film may be formed by the method in which after the polymer solution, etc. is coated in the form of a thin film, a solution of polyvalent metal salt is sprayed on the film, the method involves very complicated processes so that the efficiency of work cannot be increased as one may desire.

In addition, unwoven fabric is opaque white or something like that and when it is applied to face, hand, arm, etc., it can be immediately recognized by other persons that it is applied. As a result, if users want to go out from home, the packing agent must be removed and hence time and place for application are limited. In particular, when a gel sheet is used in order to obtain whitening and thinning effects, it is important that it be applied to the skin for a long time and in addition it is frequently applied to portions that are attractive such as hands, arms, legs, etc. In this regard, it is preferred that the hydrous gel sheet itself be as unattractive as possible.

On the other hand, when importance is placed on transparency of the sheet, use of a transparent plastic film such as a polyethylene film or a polyvinyl chloride film may be considered. However, such a plastic film is poor in interlocking with the hydrous gel layer so that when the sheet is peeled off, a so-called residual gel phenomenon occurs, that is, the gel remains on the surface of the skin after the peeling of the hydrous gel sheet, and a countermeasure against it is necessary.

### SUMMARY OF THE INVENTION

The present invention has been accomplished with view to obviating the above-described problems and an object of the present invention is to efficiently provide a gel sheet for cosmetics in the form of a thin film which is excellent in giving a feeling upon application.

Another object of the present invention to provide a gel sheet for cosmetics which has increased interlocking with a substrate and does not cause strike through.

Still another object of the present invention is to provide a gel sheet for cosmetics that is transparent and unattractive upon application to a skin.

Yet another object of the present invention is to provide a method for producing a gel sheet for cosmetics efficiently.

As a result of intensive study, the present inventors have found that at least one of the above-described objects can be attained by use of a substrate having a specified structure, thus completing the present invention.

In a first aspect, the present invention provides the following gel sheets for cosmetics.
1) A gel sheet for cosmetics, comprising a sheet-like substrate having laminated thereon a hydrous gel sheet, wherein the substrate comprises a gel non-impregnating layer and a gel impregnating layer and wherein the hydrous gel layer is provided on the gel non-impregnating layer.
2) The gel sheet for cosmetics as described in 1) above, wherein the hydrous gel layer is in the form of a thin film.
3) The gel sheet for cosmetics as described in 1) above, wherein the gel non-impregnating layer is a hydrophobic film or foamed sheet.
4) The gel sheet for cosmetics as described in 1), 2) or 3) above, wherein the gel non-impregnating layer is a transparent film.
5) The gel sheet for cosmetics as described in 1), 2), 3) or 4) above, wherein the gel impregnating layer is a fiber layer or a hydrophilic film.
6) The gel sheet for cosmetics as described in 5) above, wherein the gel impregnating layer is woven fabric, unwoven fabric or paper.
7) The gel sheet for cosmetics as described in 3) or 4) above, wherein the film of the gel non-impregnating layer is perforated
8) The gel sheet for cosmetics as described in 3) or 4) above, wherein the film of the gel non-impregnating layer is a polyurethane film.
9) The gel sheet for cosmetics as described in 1), 2), 3), 4), 5), 6), 7) or 8) above, wherein the gel impregnating layer contains a carboxyvinyl polymer and is crosslinked with a polyvalent metal salt.
10. The gel sheet for cosmetics as described in 1), 2), 3), 4), 5), 6), 7), 8) or 9), wherein the sheet-like substrate contains a whitening component.
11) The gel sheet for cosmetics as described in 1) above, wherein the whitening component is at least one substance selected from vitamin C or derivatives thereof, vitamin E nicotinate, hydroquinone, ellagic acid, albumin and galenical extracts.
12) A method for producing a gel sheet for cosmetics, comprising the steps of:
   mixing and stirring a solution or suspension of a carboxyvinyl polymer with a polyvalent metal salt and forming a hydrous gel layer in the form of a thin film using a coater; and
   winding the resulting film into a roll and heating the roll for aging.
13) The method for producing a gel sheet for cosmetics as described in 12) above, further comprising the steps of:
   forming the hydrous gel layer on a separator;
   applying a substrate onto the hydrous gel layer thus formed to form a composite; and
   winding the composite into a roll.
14) The method for producing a gel sheet for cosmetics as described in 12) above, further comprising the steps of:
   forming the hydrous gel layer on the substrate;
   applying a separator onto the hydrous gel layer thus formed to form a composite; and
   winding the composite into a roll.
15) The method for producing a gel sheet for cosmetics as described in 14) above, wherein the substrate comprises a gel non-impregnating layer and a gel impregnating layer and wherein the hydrous gel layer is laminated on the gel impregnating layer.
16) The method for producing a gel sheet for cosmetics as described in 14) above, further comprising the step of:
   punching the substrate and/or hydrous gel layer to have a predetermined shape before being wound into a roll.
17) The method for producing a gel sheet for cosmetics as described in 15) above, further comprising the step of:
   punching the substrate and/or hydrous gel layer to have a predetermined shape before being wound into a roll.

The above and other objects, effects, features and advantages of the present invention will become more apparent from the following description of preferred embodiments.

### DETAILED DESCRIPTION OF THE INVENTION

Herein, the term "gel impregnating layer" means a layer which a hydrous gel made of, for example, polyacrylic acid or salts thereof can penetrate partially or entirely and Examples thereof include fiber layer made of hydrophilic woven fabric or unwoven fabric, made of pulp (paper), rayon, cotton, etc., hydrophobic woven fabric or unwoven fabric made of polypropylene or polyester, or hydrophilic film made of polyvinyl alcohol, pullulan, etc.

By forming a hydrous gel layer on the gel-impregnating layer, the hydrous gel penetrates into a part or whole of the gel-impregnating layer to increase interlocking between the gel-impregnating layer and the hydrous gel layer laminated thereon. As a result, the hydrous gel layer immediately after its formation into a thin film on the substrate can be wound into a roll without increasing the crosslinking rate of the hydrous gel itself.

On the other hand, the term "gel non-impregnating layer" means a layer which a hydrous gel cannot penetrate and examples thereof include films of polyolefins such as polyethylene and polypropylene, various types of hydrophobic films such as flexible polyvinyl chloride film, polyester film, nylon film, and polyurethane film, and closed cell foamed films made of, for example, polyethylene, polyurethane, various types of rubber such as SBR (styrene block copolymers). In particular, use of polyurethane films, more particularly, films of polyether polyamide, polyether polyurethane, polyester polyurethane, polyether polyester or ethylene/vinyl acetate copolymer, increases the stretchability of the gel non-impregnating layer so that a hydrous gel sheet including a gel non-impregnating layer made of polyurethane films having such an increased stretchability is advantageous for use in applying to the skin of such sites of the body which moves, e.g., being bent or stretched, e.g., elbow, knee, etc. In the case where ethylene/vinyl acetate copolymers are used, it is preferred that they have a vinyl acetate content of 20% by weight or more.

The gel non-impregnating layer, which constitutes an outer layer of the substrate, has an effect of preventing the strike through of the hydrous gel formed into a film on the gel impregnating layer. As a result, when the substrate on which the hydrous gel layer is formed is wound into a roll and stored, there occurs no strike through of the hydrous gel layer and the thickness of the hydrous gel formed on the gel impregnating layer can be maintained at a constant value. This minimizes the fluctuation in thickness of the hydrous gel when it is formed into a thin film.

As a substrate exhibiting such an effect can be used any combination of the above-mentioned gel non-impregnating layer and gel impregnating layer. More particularly, there can be used advantageously laminates of polyethylene film/pulp (paper), polyester film/rayon unwoven fabric, polyester film/polyester unwoven fabric, polypropylene film/polyvinyl alcohol film, polyurethane film/polypropylene unwoven fabric, SBR foamed film/polyester unwoven fabric, etc.

In this case, use of pulp or rayon as the fiber for the fiber layer as a gel impregnating layer and a transparent film as a gel non-impregnating layer provides a sheet for cosmetics giving an impression of transparency. It is considered that such a constitution allows the moisture contained in the hydrous gel layer to penetrate the fiber, with the result that the amount of light scattered by the fiber decreases to make the gel sheet for cosmetics applied to the skin unattractive.

The gel sheet for cosmetics of the present invention is not intended to make the hydrous gel sheet itself to have transparency but to make it unattractive that a hydrous gel sheet is present when it is applied to the skin surface so that an increase in light transmittance is not always intended. That is, the reason that transparency or impression of being transparent can be obtained is considered to be that as described above, not only the light scattered by the fiber layer decreases in amount but also the application of a hydrous gel sheet to the skin surface decreases scattering of light on the surface of the hydrous gel layer where the sheet is applied to the skin. Therefore, it is considered that in the present invention, similar effects can be obtained by use of water absorbing fibers other than pulp or rayon, for example, cupra, acetate rayon, etc.

It is preferred that paper or unwoven fabric produced by a dry method or a wet method be used as a fiber layer. Use of knitted web or woven fabric made of a pulp or rayon fiber would be considered possible. However, this is not preferable in that in this case there will be a high contrast between the fiber material used for knitted web or woven fabric and the surroundings, so that the existence of fiber material will become conspicuous.

Pulp and rayon may be used singly or in combination for constituting the fiber layer. In the case where a layer of pulp or rayon alone is used, it is sometimes the case that the layer is weak in mechanical strength and in order to reinforce it, other synthetic fibers such as those made of polyester, for example, may be mixed with pulp or rayon so long as the effect of the present invention is not adversely affected. If the other synthetic fibers are mixed in a high proportion in the mixture, the above-described purpose is not attained and, hence, they are used such that the proportion of pulp or rayon in the total weight of fiber layer is generally 50% by weight or more, preferably 70% or more.

In the substrate, the gel impregnating layer that can be used advantageously has a basis weight of 5 to 60 g/m², preferably 10 to 30 g/m², for an unwoven fabric substrate, or a thickness of 5 to 100 µm for a film substrate. The gel non-impregnating layer that can be used advantageously has a thickness of 1 to 100 µm for an ordinary or non-foamed plastic film or 0.3 to 1 mm for a foamed plastic film. Those films having the basis weight or film thickness lower than the above tend to exhibit the above-described functions insufficiently while those films having the basis weight or film thickness higher than the above tend to give an aggravated thickness impression. For example, in the case of a gel sheet for cosmetics giving an impression of transparency, the penetration of moisture into the fiber layer plays an important role. In particular, in the aspect of material, use of a hydrophilic fiber such as pulp or rayon results in good penetration of moisture. It is desirable that moisture penetrates the fiber layer throughout to spread substantially in all the thickness direction. If the basis weight of the gel impregnating layer exceeds 60 g/m², moisture does not penetrate all the layer or if it penetrates, it is possible that the gel sheet fails to give an impression of transparency. On the other hand, if the gel impregnating layer has a basis weight of less than 5 g/m², its thickness is small so that no sufficient interlocking can be obtained. This does not mean that the hydrous gel penetrates all over the fiber layer throughout but the gel impregnating layer may be impregnated with the hydrous gel layer only partially. In other words, it is simply required that moisture penetrates the fiber layer throughout (which means all over the plane region on which the hydrous gel layer is laminated). Furthermore, in view of reduction in thickness of the product, a substrate having a total thickness of 1 mm or less, preferably 500 µm or less, is used advantageously. If the thickness of the substrate exceeds 1 mm in total, the thickness impression is worse and the gel sheet tends to be readily peeled off after its application to the skin.

On the other hand, when importance is posed on transparency, a transparent film as a gel non-impregnating layer is laminated on an outer surface of the fiber layer as a gel impregnating layer. As described above, in the present invention, the fiber layer (gel impregnating layer) constituting the substrate is susceptible to the penetration of moisture and in order to give an impression of transparency, use is made of a hydrophilic material such as pulp or rayon for a fiber layer. For this reason, if the substrate is constituted by the fiber layer alone, the substrate not only fails to exhibit its function as a substrate sufficiently but also causes strike through. In this regard, the films, i.e., a gel non-impregnating layer, is laminated on the fiber layer.

In the present invention, the substrate can be obtained by laminating a gel impregnating layer and a gel non-impregnating layer. In this case, the method for obtaining the substrate is not limited particularly and, for example, a dry lamination method, and various types of extrusion lamination methods may be used.

The gel sheet of the present invention can be used for cosmetics and sometimes a cool feeling upon its application to the skin surface is desired. In this case, it is desirable that the evaporation of moisture from the backside surface of the gel sheet be accelerated in order to prolong a feeling of coolness. When a film is used as the gel non-impregnating layer of the substrate, moisture is prevented from evaporating from the gel non-impregnating layer. As a result, the feeling of coolness does not last. Therefore, it is preferred to provide the film of gel non-impregnating layer with a number of perforations so long as the hydrous gel layer causes no strike through. The size of the perforations is not limited particularly but generally it is preferred that perforations having a diameter of about 50 to about 2,000 µm, preferably 200 to 1,000 µm, in a proportion of about 1 to about 100 perforations/cm², preferably about 5 to about 50 perforations/cm².

The hydrous gel layer is not limited particularly so long as it can be extended into a sheet-like shape before use. For example, those obtained by crosslinking various water-soluble polymers with polyvalent metal salts, or those crosslinked by any other crosslinking means such as radiation crosslinking can be used. In particular, in order to form a hydrous gel layer in the form of a thin film-like shape, water-soluble polymers containing a carboxyvinyl polymer and crosslinked with a polyvalent metal salt is preferably used.

Here, The term "carboxyvinyl polymer" as referred to herein is used in a broader sense to include polymers of an isomer or isomers having a carboxyvinyl group, such as polyacrylic acid or polyacrylates and partially or totally neutralized derivatives thereof and further partially crosslinked ones. This term represents a concept that covers also a carboxyvinyl polymer in a narrower sense which is a copolymer comprising acrylic acid as a major component to which a small amount of allyl sucrose is blended, and a part of which is crosslinked, such as those commercially available products under trade name Carbopol (a product of B.F. Goodrich Chemical Company), High Vis Wako (a product of Wako Pure Chemical Industries, Ltd.), Junron (a product of Nippon Junyaku Co., Ltd.), etc.

The carboxyvinyl polymer includes, for example, polyacrylic acid and/or salts thereof. It is desirable to use polyacrylic acid and/or salts thereof having a weight average molecular weight of 20,000 to 10,000,000, preferably 1,000,000 to 7,000,000. It is also desirable to adjust the hydrous gel layer to contain them in a concentration of about 2 to about 40% by weight, preferably 3 to 10% by weight. Use of the carboxyvinyl polymer having a weight average molecular weight in the above-described range in a concentration in the above-described range results in producing an optimal three-dimensional skeleton.

Further, in the present invention, it is preferred to blend polyhydric alcohols. The polyhydric alcohol which may be contained in the hydrous gel layer includes glycols such as ethylene glycol, diethylene glycol, triethylene glycol, polyethylene glycol, and propylene glycol, polypropylene glycol, diols such as 1,3-propanediol and 1,4-butanediol, etc. as well as glycerin and sorbitol. Out of these, use of glycerin is preferred mainly because it has excellent moisture retention ability and there have been known ample precedent examples for its use and its safety has been already confirmed.

The polyvalent alcohols act as a plasticizer for the polyacrylic acid, polyacrylic acid salts, etc. and in addition they retain moisture to exhibit humectant effect. Their content is generally 5 to 90% by weight, preferably 10 to 80% by weight, in the hydrous gel layer. The content of glycerin is usually 10 to 50% by weight but is not limited thereto.

The water contained in the hydrous gel layer is an important component for moisturizing and hydrating the site of skin where the gel sheet is applied to. It is desirable that the water content in the hydrous gel layer be adjusted to 10% by weight or more, preferably in the range of 30 to 70% by weight. If the water content is less than 10% by weight, the effect of hydrating the skin may sometimes be insufficient.

Furthermore, externally applied crosslinking agent to be added for crosslinking the polyacrylic acid or polyacrylic acid salts includes polyvalent metal salts, polyvalent metal hydroxides, and polyvalent metal oxides, such as aluminum hydroxide, potassium alum, aluminum sulfate, aluminum glycinate, aluminum acetate, aluminum oxide, aluminum metasilicate, magnesium chloride, aluminum hydroxide, and calcium carbonate. Also, epoxy compounds may be used.

The blending amount of the crosslinking agents may vary depending on the kind of crosslinking agent but it is preferred that the blending amount be set to usually 0.1 to 5% by weight based on the total weight of composition. If the blending amount of the crosslinking agent is too small, crosslinking occurs insufficiently so that the polyhydric alcohols, water and other components can be retained only insufficiently and the mechanical strength of the gel layer itself decreases. On the other hand, if the blending amount of the crosslinking agent is too large, the crosslinking proceeds too much to make the gel layer stiff and less flexible and the retention of other components tends to become insufficient.

Of course, the hydrous gel layer may contain in addition to the above-described substances, various water-soluble polymers, various inorganic or organic fillers such as silica, zinc oxide, calcium carbide, hydroxylapatite, and carbon, perfumes, various plant and animal extracts (liquid) , such as aloe extracts (liquid), Hamamelis extract (liquid), hyaluronic acid, refreshers such as 1-menthol, antioxidant such as tocopherol acetate, BHT (di-tert-butyl-p-cresol), mildewproofing agents such as paraoxybenzoic acid or esters thereof, etc. may be blended. In addition to these, those additives commonly used in cosmetics, such as whitening agents, anti-crease agent, various ultraviolet absorbents, etc. may be blended regardless of whether or not such are active components. Further, the pH of the hydrous gel layer can be adjusted with citric acid, tartaric acid, triethanolamine, etc. to adjust the crosslinking rate or the solubility of various additives.

By blending various keratin softeners, cell activating components, oil components, whitening components, etc. in the hydrous gel layer, symptoms such as keratosis of various sites such as elbow, knee, calx, and ankle, dry skin, hypertrophic keratosis, coarse skin, and chap (creasing) can be improved or the gel sheet can be utilized for sheet for cosmetics for skin care where a long term application of the sheet is required, such as whitening cosmetics.

Further, if needed, perfumes, antiseptic/mildewproofing agents, colorants, etc. may be blended conveniently.

Furthermore, where an increase in the activity of whitening components to penetrate into the skin when the gel sheet is applied, or oil-soluble components are blended in the hydrous gel layer, surfactants may be added in the hydrous gel layer in advance. As such a surfactant, there can be used one or more of cationic surfactants such as quaternary ammonium slats and alkylpyridinium salts, anionic surfactants such as sodium alkylbenzenesulfonate and sodium dodecylsulfonate, nonionic surfactants such as polyoxyethylene alkyl ether and fatty acid esters of sucrose, amphoteric surfactants such as N-alkyl-N,N-dimethylammonium betaine.

In the present invention, the whitening component to be blended in the hydrous gel layer is not limited particularly so long as it exhibits whitening effect by preventing deposition of or removing the pigments in the skin. More particularly, there can be cited those substances which have an effect of inhibiting the generation of melanine which causes the deposition of pigments, those substances which have an effect of improving the moisture retention of corneum of the skin, those substances which improves blood circulation in the skin in order to remove darkness, those substances which have an effect of removing corneum containing melanine, etc. Specifically, vitamin C and its derivatives, vitamin E nicotinate, hydroquinone, ellagic acid, albumin, and galenical extracts (chamomile extracts, saxifrage (Saxifraga stolonifera Meerb.) extracts, scuttellaria root extracts, etc.) are preferred. It is effective to blend at least one out of them.

The content of the whitening component described above is not limited particularly so long as it exhibits whitening effect. Usually, addition of it in the hydrous gel layer in an amount on the order of about 0.1 to about 10% by weight, preferably 0.2 to 5% by weight can give a sufficient whitening effect.

The whitening component may be added in the form of a substance itself or in the form of a whitening cosmetic such as beauty wash or cream containing a whitening component. In particular, in the case of liquid preparations, the latter form is advantageous since it is highly compatible with the water-soluble polymer constituting the hydrous gel layer and can be homogeneously mixed therewith without difficulty during its production.

The whitening component can be blended into the hydrous gel layer as described above. However, the method of blending it is not limited thereto and it can also be provided on the substrate by mixing it with a water-soluble polymer and water and/or a hydrophilic medium such as an alcohol homogeneously to prepare an application layer composition (liquid), and coating the resulting preparation on a surface of a separator or on a sheet-like substrate without drying or subsequently drying the coated film to form an application layer. It should be noted that when the application layer is formed by drying, it is unnecessary to completely remove the water or hydrophilic medium but from several percents by weight to several tens percents by weight of them may remain in the coated film.

The above whitening component may be blended with a water-soluble composition, polyhydric alcohol, water, or the like and blended in the hydrous gel. Alternatively, beauty wash or lotion containing the whitening component may be coated on the skin amply and onto the thus coated skin the gel sheet for cosmetics of the present invention may be applied to provide whitening effect.

The gel sheet for cosmetics of the present invention may be subjected to release treatment in advance by coating a releasing agent such as silicone on the back side of the substrate, that is, on the side of the gel non-impregnating layer.

Further, the gel sheet for cosmetics of the present invention is cut to a predetermined size before it can be shipped. Therefore, in this case, a separator is applied on an exposed surface in order to protect the exposed surface of the hydrous gel layer from dust or foreign matter. The separator is not limited particularly and polyethylene films, polypropylene films, polyester films, etc. may be used. By applying a separator in this manner, the gel sheet of the present invention can be wound into a roll without performing release treatment in advance. Of course, it is more preferable that release treatment is effected on the surface of the separator on which the hydrous gel layer is applied.

The gel sheet for cosmetics can be produced by a conventional method. For example, after a hydrous gel layer is formed on a separator, it is transferred onto a surface of a fiber layer on the substrate and then wound into a roll, which is stored at room temperature or under heating to complete crosslinking. Alternatively, a hydrous gel layer may be formed on the surface of a fiber layer of the substrate followed by completion of crosslinking. Furthermore, the substrate and/or hydrous gel layer may be punched to have a predetermined shape before being wound into a roll.

The gel sheet for cosmetics of the present invention is produced preferably by the following method.

That is, the gel sheet for cosmetics of the present invention can be produced by a method comprising the steps of mixing and stirring a solution or suspension of a carboxyvinyl polymer with a polyvalent metal salt and forming a hydrous gel layer in the form of a thin film using a coater and winding the resulting film into a roll and optionally heating the roll for aging.

That is, in the present invention, a gel non-impregnating layer is provided on an outer surface of a gel impregnating layer after forming a hydrous gel layer thereon and the hydrous gel layer is formed in the form of a thin layer and immediately thereafter, the composite is wound into a roll, followed by heating at room temperature or higher temperatures for aging to complete crosslinking. First, the carboxyvinyl polymer (water-soluble polymer) such as polyacrylic acid is dissolved or suspended in water or an aqueous alcohol solution. Then, additives such as various fillers and perfumes, various animal and plant extracts are added thereto and the mixture was stirred uniformly. Thereafter, a polyvalent metal salt is added and stirred and the resulting mixture is coated on the substrate to form a thin film using a coater. As the coater, there is, for example, a coater which is used for the production of adhesive tapes as it is. For example, such a coater includes various roll coaters represented by a comma coater and various blade coaters represented by a fountain blade coater.

In this case, the hydrous gel layer is coated in the form of a thin film, that is, to a hydrous gel layer thickness of 2 mm or less, preferably 1,000 µm or less, and more preferably 50 to 500 µm or less. After coating the hydrous gel layer in is not limited. Fiber layer made of pulp or rayon is white or of a color similar to white and use of colorless material as a transparent film gives a more excellent impression of transparency. However, the color of skin is diversified and there may be a need for distinguishing the color of the gel sheet for cosmetics depending on its utility or the kinds of blended components therein. For this purpose, the hydrous gel, fiber layer or transparent film may be colored lightly to an extent such that the impression of transparency is not damaged.

### EXAMPLES

Hereafter, the present invention will be described in more detail by examples, production examples, comparative examples, and reference example. However, the present invention is not limited thereto.

### Production Example 1

Sodium polyacrylate (6 parts by weight), 4 parts by weight of crosslinked polyacrylic acid, and 0.1 part by weight of paraoxybenzoic acid ester were suspended in 40 parts by weight of glycerin and the suspension was blended with 50 parts by weight of purified water and 0.3 part by weight of dry aluminum hydroxide to obtain a gel-like composition. This was coated on a silicone-treated surface of a polyester film of 30 cm in width to a gel thickness of about 1 mm by using a bar coater and various substrates as shown in Table 1 were each applied to the resulting film. The composite was immediately wound into a roll of 30 m long continuously. Thereafter the roll was sealed with an aluminum sheet and aged at 50°C for 3 days to obtain gel sheets for cosmetics of Examples 1 to 4 and Comparative Examples 1 to 3. The substrates were applied so that their respective unwoven or pulp surfaces contacted the hydrous gel layer. Various gel sheets for cosmetics thus obtained were examined on the degree of completion of gelling and occurrence of strike through. The the form of a thin film on the substrate, optionally a separator is applied and the composite, as it is, is wound into roll and then sealed with a plastic, if needed, heated to about 50°C for storage to effect crosslinking to obtain a final product.

Alternatively, after forming a hydrous gel layer directly on a separator by use of a coater, the substrate is applied thereto and the resulting composite is wound into a roll as it is. In this case, too, it is preferred that the hydrous gel layer be formed into a thin film of 2 mm or less thick. Of course, it may be formed into a thick film, for example, by using a conventional coater.

As described above, in the present invention, use is made of a two-layer substrate comprising a gel impregnating layer and a gel non-impregnating layer and a hydrous gel layer is formed in the form of a thin film so that the composite can be immediately wound into a roll and crosslinking is completed.

In particular, according to the present invention, it is convenient in that it is possible to form a hydrous gel layer in the form of a thin film on the substrate, etc. directly by using a coater, and after the coating on the substrate, the resulting film can be directly wound into a roll without preliminary drying. Therefore, while preventing strike through of the hydrous gel, a gel sheet for cosmetics of the type of a thin film with a minimized fluctuation in thickness can be produced efficiently. Furthermore, since no preliminary drying is performed, there occurs no volatilization of perfumes or the like during the production process so that a merit is obtained that volatile additives such as 1-menthol can be added.

As described above, the present invention makes it possible to give an impression of transparency and render the gel sheet for cosmetics unattractive or conspicuous when applied to the skin by use of a transparent film as the gel non-impregnating layer. Its field of application, however, results are shown in Table 1. As will be apparent from Table 1, the gel sheets for cosmetics of Examples gelled sufficiently and showed no strike through.

Evaluation of gelling was made according to the following criteria:
○: No gelling was observed visually.
X: Gelling was observed visually.

Evaluation of the occurrence of strike through was made based on the following criteria:
○: No penetration of hydrous gel layer to the back side of the substrate was observed visually.
X: Penetration of hydrous gel layer to the back side of the substrate was observed visually.

**Table 1**

| | Substrate (Gel non-impregnating layer/gel impregnating layer) | Gelling | Strike through |
|---|---|---|---|
| Example 1 | Polyester film 6 µm /polyester unwoven fabric 20 g/m² | ○ | ○ |
| Example 2 | Polyurethane film 30 µm /rayon unwoven fabric 30 g/m² | ○ | ○ |
| Example 3 | Polyethylene film 20 µm /pulp 30 g/m² | ○ | ○ |
| Example 4 | SBR foamed film 300 µm /polyester unwoven fabric 50 g/m² | ○ | ○ |
| Comparative Example 1 | Polyester unwoven fabric 40 g/m² | ○ | X |
| Comparative Example 2 | Rayon unwoven fabric 40 g/m² | ○ | X |
| Comparative Example 3 | Polypropylene unwoven fabric 50 g/m² | ○ | X |

### Production Example 2

Sodium polyacrylate (6 parts by weight), 4 parts by weight of crosslinked polyacrylic acid, and 0.1 part by weight of paraoxybenzoic acid ester were suspended in 40 parts by weight of glycerin and the suspension was blended with 50 parts by weight of purified water and 0.3 part by weight of dry aluminum hydroxide to obtain a gel-like composition. This was coated on a silicone-treated surface of a polyester film of 30 cm in width to a gel thickness of about 1 mm by using a bar coater and immediately thereafter various substrates as shown in Table 2 were each applied to the resulting film. The composite was wound into a roll of 30 m long continuously. Thereafter the roll was sealed with an aluminum sheet and aged at 50°C for 3 days to obtain gel sheets for cosmetics of Examples 5 to 7 and Comparative Example 4 and Reference Example 1. The substrates were applied so that their respective unwoven or pulp surfaces contacted the hydrous gel layer. Various gel sheets for cosmetics thus obtained were examined on the occurrence of strike through and interlocking (residual gel upon peeling). The results are shown in Table 2. As will be apparent from Table 2, the gel sheets for cosmetics of Examples showed no strike through and gave an excellent interlocking effect and excellent impression of transparency. The gel sheet for cosmetics of Reference Example 1 showed no strike through and exhibited satisfactory interlocking effect but gave an insufficient impression of transparency as a gel sheet for cosmetics intended to give an impression of transparency when applied to the skin.

**Table 2**

| | Substrate (Gel non-impregnating layer /gel impregnating layer) | Strike through | Interlocking | Transparency |
|---|---|---|---|---|
| Example 5 | Polyethylene film 20 µm /pulp 15 g/m² | ○ | ○ | Transparent |
| Example 6 | Polyethylene film 20 µm/rayon unwoven fabric 20 g/m² | ○ | ○ | Slightly transparent |
| Example 7 | Polyurethane film 30 µm/rayon 30 g/m² | ○ | ○ | Slightly transparent |
| Comparative Example 4 | Polyester unwoven fabric 40 g/m² | X | ○ | Transparent |
| Reference Example 1 | Polyester film 6 µm 40 g/m² | ○ | ○ | Nontransparent |

### Production Example 3

Sodium polyacrylate (6 parts by weight), 4 parts by weight of crosslinked polyacrylic acid, and 0.1 part by weight of paraoxybenzoic acid ester were suspended in 40 parts by weight of glycerin and the suspension was blended with 50 parts by weight of purified water and 0.3 part by weight of dry aluminum hydroxide to obtain a gel-like composition. This was coated on a silicone-treated surface of a polyester film of 30 cm in width to a gel thickness of about 1 mm by using a bar coater. Then a two-layer substrate comprising a 20 µm thick polyethylene film provided with 2,000/m² of perforations (each about 300 µm in diameter) and a pulp layer in a basis weight of 15 g/cm² was applied to the resulting film so that the pulp surface of the substrate contacted the hydrous gel layer. The composite was immediately wound into a roll of 30 m long continuously. Thereafter the roll was sealed with an aluminum sheet and aged at 50°C for 3 days to obtain a gel sheet for cosmetics of Example 8. In this gel sheet for cosmetics, gelling occurred sufficiently but no strike through was observed.

Then, the gel sheet for cosmetics of the instant Example with perforations and that of Example 3 provided with no perforation were each cut to a size of 5 cm × 7 cm and the specimens were applied to a calf and a feeling of coolness was examined. As a result, no coolness was felt after about 5 minutes for the gel sheet without any perforation whereas coolness was continued to be felt for about 20 minutes for the gel sheet with perforations. The both gel sheets showed good adhesion to the skin and no gel remained on the skin when it was peeled off. The tests were performed by a panel of 10 female volunteers of 20 to 30 ages and judgment was made when 8 or more panelists agreed.

The gel sheet for cosmetics of the present invention, which has the constitution that a hydrous gel layer is formed on a two layer substrate which comprises a gel impregnating layer and a gel non-impregnating layer, does not show strike through when the resulting composite is wound into a roll and aged for the completion of crosslinking to gives rise to a gel sheet for cosmetics having a minimized fluctuation in thickness.

Further, by coating a hydrous gel layer in the form of a thin layer, the hydrous gel layer can be formed on a substrate without preliminary drying and immediately wound into a roll. As a result, the gel sheet of the invention can be produced by a generally used coater as used commonly in the production of adhesive tapes so that the efficiency of operation can be increased remarkably. In addition, the strike through of the hydrous gel layer and fluctuation in thickness of the hydrous gel layer can be alleviated or minimized.

The gel sheet for cosmetics of the present invention in a preferred embodiment has a substrate comprising a fiber layer made of pulp or rayon and a transparent film laminated thereon and a hydrous gel layer on the fiber layer of the substrate and, hence, it can give an impression of increased transparency when it is applied to the skin and make one feel as if there were applied no such a sheet on a first sight.

## Claims

1. A gel sheet for cosmetics, comprising a sheet-like substrate having laminated thereon a hydrous gel sheet, wherein said substrate comprises a gel non-impregnating layer and a gel impregnating layer and wherein said hydrous gel layer is provided on said gel non-impregnating layer.

2. The gel sheet for cosmetics as claimed in claim 1, wherein said hydrous gel layer is in the form of a thin film.

3. The gel sheet for cosmetics as claimed in claim 1, wherein said gel non-impregnating layer is a hydrophobic film or foamed sheet.

4. The gel sheet for cosmetics as claimed in claim 1, 2 or 3, wherein said gel non-impregnating layer is a transparent film.

5. The gel sheet for cosmetics as claimed in claim 1, 2, 3 or 4, wherein said gel impregnating layer is a fiber layer or a hydrophilic film.

6. The gel sheet for cosmetics as claimed in claim 5, wherein said gel impregnating layer is woven fabric, unwoven fabric or paper.

7. The gel sheet for cosmetics as claimed in claim 3 or 4, wherein the film of said gel non-impregnating layer is perforated

8. The gel sheet for cosmetics as claimed in claim 3 or 4, wherein the film of said gel non-impregnating layer is a polyurethane film.

9. The gel sheet for cosmetics as claimed in claim 1, 2, 3, 4, 5, 6, 7 or 8, wherein said gel impregnating layer contains a carboxyvinyl polymer and is crosslinked with a polyvalent metal salt.

10. The gel sheet for cosmetics as claimed in claim 1, 2, 3, 4, 5, 6, 7, 8 or 9, wherein said sheet-like substrate contains a whitening component.

11. The gel sheet for cosmetics as claimed in claim 1, wherein the whitening component is at least one substance selected from vitamin C or derivatives thereof, vitamin E nicotinate, hydroquinone, ellagic acid, albumin and galenical extracts.

12. A method for producing a gel sheet for cosmetics, comprising the steps of:
mixing and stirring a solution or suspension of a carboxyvinyl polymer with a polyvalent metal salt and forming a hydrous gel layer in the form of a thin film using a coater; and
winding the resulting film into a roll and heating the roll for aging.

13. The method for producing a gel sheet for cosmetics as claimed in claim 12, further comprising the steps of:
forming said hydrous gel layer on a separator;
applying a substrate onto the hydrous gel layer thus formed to form a composite; and
winding the composite into a roll.

14. The method for producing a gel sheet for cosmetics as claimed in claim 12, further comprising the steps of:
forming said hydrous gel layer on said substrate;
applying a separator onto the hydrous gel layer thus formed to form a composite; and
winding the composite into a roll.

15. The method for producing a gel sheet for cosmetics as claimed in claim 14, wherein said substrate comprises a gel non-impregnating layer and a gel impregnating layer and wherein said hydrous gel layer is laminated on said gel impregnating layer.

16. The method for producing a gel sheet for cosmetics as claimed in claim 14, further comprising the step of:
punching said substrate and/or hydrous gel layer to have a predetermined shape before being wound into a roll.

17. The method for producing a gel sheet for cosmetics as claimed in claim 15, further comprising the step of:
punching said substrate and/or hydrous gel layer to have a predetermined shape before being wound into a roll.
